# EUROPEAN PATENT APPLICATION

(11) **EP 2 719 455 A1**
(43) Date of publication of application: **16.04.2014**
(21) Application number: 12382387.4
(22) Date of filing: 09.10.2012
(51) Int. Cl.: B01J 23/46, B01J 37/02, C07C 5/10, B01J 31/02

(54) **A compound comprising ruthenium nanoparticles supported on a porous solid support for the hydrogenation of aromatic compounds.**

(71) Applicant: Fundació Institut Català d'Investigació Química, 43007 Tarragona (ES)
(72) Inventor: van Leeuwen, Petrus Wilhelmus Nicolaas Maria, 3628CA Kockengen (NL); Gonzáles Gálvez, David, 08224 Terrassa (ES)
(74) Representative: ZBM Patents - Zea, Barlocci & Markvardsen

(57) **Abstract**

The present invention relates to a process for preparing a catalyst comprising ruthenium nanoparticles supported on a solid support, obtainable by: (a) reacting a ruthenium (0) complex selected from the group consisting of [Ru(diene)(triene)], [Ru(R₆)(diene)], and Ru₃(CO)₁₂, with a phosphine ligand of formula (I) in the presence of hydrogen; and (b) reacting the ruthenium nanoparticles obtained in Step (a) with a porous solid support, and to the obtained supported phosphine-ruthenium nanoparticles. It is also related to a process for hydrogenating an aromatic compound into the corresponding cyclic saturated or partially unsaturated compound by reaction with hydrogen and in the presence of an effective amount of the obtained supported ruthenium nanoparticles.

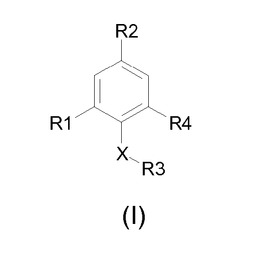

## Description

The present invention relates to the field of supported ruthenium catalysts. In particular, it relates to a process for the preparation of a compound comprising phosphine-ligated ruthenium nanoparticles supported on a solid support, to the catalyst obtained by this process, and to a process for hydrogenating aromatic compounds using this catalyst.

### BACKGROUND ART

The complete hydrogenation of aromatic compounds is one of the most important and challenging transformations in the synthesis of fine chemicals and intermediates. It is interesting in the petrochemical industry and in the generation of clean diesel fuels.

It is known that several dissolved metal nanoparticle catalysts have been used to hydrogenate aromatic compounds such as benzene and some of its derivatives. The use of these dissolved catalysts involves the tedious separation of the expensive transition-metal catalyst from the substrates and products and the need to recycle the catalyst, thereby remaining an important drawback for their industrial application.

Furthermore, the use of metallic nanoparticles (NPs) finely dispersed in water has proved to be a sustainable alternative to conventional homogeneous catalysts. Nanoparticles usually provide higher activity than other compounds having a high active surface area. The main advantage of the nanoparticles in catalytic applications is linked to their high specific surface area resulting in a high number of potential catalytic sites as well as the possibility to tune their surface properties, which could greatly improve their specific catalytic activity. Unfortunately, the recycling of nanoparticles implies an additional step of nano-filtration or decantation that is difficult and expensive to implement industrially.

Ruthenium (0) nanoclusters stabilized by a nanozeolite framework have been disclosed as colloidal catalysts for the hydrogenation of neat aromatics under mild conditions. Those catalysts provide a turnover number up to 5420 h⁻¹ at room temperature in the hydrogenation of neat benzene with a good conversion. When reused, the catalyst retains 92% of its initial catalytic activity for the third reaction run. Unfortunately, these colloidal catalysts need to be handled under dry and inert atmosphere, which hinder their industrial implementation (Özkar, et al. "Ruthenium (0) nanoclusters stabilized by a nanozeolite framework: isolable, reusable, and green catalyst for the hydrogenation of neat aromatics under mild conditions with the unprecedented catalytic activity and lifetime", Journal of the American Chemical Society, 2010, vol. 132, pp. 6541-6549).

A huge variety of compounds have been described as suitable stabilizers of metallic nanoparticles, such as for example polymers, dendrimers, ionic liquids, and chemical ligands. The incorporation of stabilizers in the metallic nanoparticles can also modify and control the size, the surface structure, and the electronic properties of the metallic nanoparticles. Unfortunately, stabilizers can also react with the active sites of the nanoparticles compromising their catalytic activity. In particular, some phosphines are known as potentially poisoning agents in catalytic reactions when catalytically active nanoparticles are used as catalysts.

A colloidal solution of ruthenium nanoparticles ligated to some specific phosphines has been disclosed for the total hydrogenation of aromatic compounds. It is mentioned that the nature of the stabilizer is crucial for the catalytic activity of the ruthenium complex. (Piet W.N.M van Leeuwen, et al. "Phosphine stabilized ruthenium nanoparticles: the effect of the nature of the ligand in catalysis", ACS Catalysis, 2012, vol. 2(3), pp. 317-321).

Unfortunately, the catalytic activity of ligated metallic nanoparticles can be compromised for their deactivation due to metal leaching and further formation of aggregates. Thus, the presence of the stabilizer does not completely allow avoiding all the disadvantages of metallic nanoparticles, such as agglomeration and ease of recycling. Therefore, immobilization of the metallic nanoparticles using biphasic systems or solid supports has been developed. In particular, metallic nanoparticles supported on a solid support have been used as catalysts, being easily separated from the organic product and therefore recovered to be reused. The catalytic activity of the metallic supported nanoparticles and/or their selectivity of the process could be modified depending on a great number of parameters such as for example the metal atom, the stabilizer, and the support used, as well as the process for their preparation.

Immobilization of small and homogeneously dispersed ruthenium nanoparticles ligated by 4-(3-phenylpropyl)pyridine ligand on solid supports such as functionalized multi-walled carbon nanotubes, silica, alumina, and activated carbon have been used in hydrogenation processes. It is noted that the nature of solid support and the interactions between the support and the metal atom are crucial for the catalytic activity, and that not all supported ligated ruthenium catalyst can be reused without a reduction of the catalytic activity. (Montserrat Gómez, et al. "Ruthenium nanoparticles supported on multi-walled carbon nanotubes: highly effective catalytic system for hydrogenation processes", Journal of Molecular Catalysis A: Chemical, 2010, vol. 332, pp. 106-112).

Therefore, from what is known in the art it is derived that there is still the need of providing catalysts for the hydrogenation of aromatic compounds having a high catalytic activity and stability.

### SUMMARY OF THE INVENTION

Inventors have found that the phosphine-ligated ruthenium compound supported on a porous solid support of the present invention does not require an initial pre-activating period, that it is more stable when handled under air, and more robust and more active than the ones known in the state of the art. Those compounds are stable under the turnover conditions allowing for a more efficient hydrogenation reaction of aromatic compounds. Unlike the behaviour of other supported ruthenium catalysts of the prior art, the catalyst of the invention does not show any sign of deactivation by aggregation or decomposition under the reaction conditions, thus maintaining the catalytic activity even after being reused 10 times. It is advantageous because the compound of the invention can be used in the presence of some well known poisoning agent, and in a continuous process.

Thus, a first aspect of the present invention relates to a process for preparing a catalyst comprising a process for preparing a compound comprising ruthenium nanoparticles supported on a porous solid support, wherein the particle size of the ruthenium nanoparticles is comprised of from 0.7 to 3 nm; and the amount of ruthenium in the ruthenium nanoparticles supported on the porous solid support is comprised of from 0.1 to 15% by weight; the process comprising: (a) reacting a ruthenium (0) complex selected from the group consisting of [Ru(diene)(triene)], [Ru(R₆)(diene)], Ru₃(CO)₁₂, and a mixture thereof, in the presence of a phosphine ligand of formula (I) or a mixture thereof 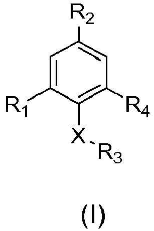 with hydrogen under an oxygen-free atmosphere and in a solvent; wherein: the molar ratio between the ruthenium (0) complex and the phosphine ligand of formula (I) is comprised of from 1:0.05 to 1:0.4; the concentration of the ruthenium (0) complex is comprised of from 0.6 to 10 mM and the reaction is carried out at a temperature comprised of from -40 to 60°C, and under a hydrogen pressure comprised of from 1 to 6 bar; and (b) reacting the ruthenium nanoparticles obtained in Step (a) with a porous solid support under an oxygen-free atmosphere and in the presence of a solvent; wherein: the weight of solid support per mole of ruthenium in the ruthenium (0) complex used in Step (a) is comprised of from 0.5 to 10 g; wherein the reaction is carried out at a temperature comprised of from -40 to 60°C; wherein: diene is selected from the group consisting of cycloocta-1,5-diene, cycloocta-1,3-diene, norbornadiene, cyclohexa-1,3-diene, and hepta-1,4-diene; triene is cycloocta-1,3,5-triene; R₁ and R₂ are independently selected from the group consisting of H, (C₁-C₁₂)alkyl, (C₁-C₆)alkoxy, and P(R₅)(R₅'), wherein one of R₁ or R₂ is P(R₅)(R₅'), and the other of R₁ or R₂ is H, (C₁-C₆)alkyl, or (C₁-C₆)alkoxy; R₃ is (C₁-C₆)alkyl; R₄ is H or (C₁-C₆)alkyl; or alternatively R₃ and R₄ in combination with the adjacent atoms form a 5 to 14-membered ring system, partially unsaturated or aromatic, which comprises from one to three 5 to 6-membered rings, the members of any of the rings being independently selected from C, CH, CH₂, O, N, and NH; being any of the rings optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, (C₁-C₆)alkoxycarbonyl, nitro, cyano, and P(R₅)(R₅'); R₅ and R₅' are independently selected from the group consisting of (C₁-C₆)alkyl, (C₃-C₇)cycloalkyl, (C₃-C₇)cycloalkenyl, (C₆-C₁₄)aryl, and (C₄-C₁₄)heteroaryl; being optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, (C₁-C₆)alkoxycarbonyl, nitro, and cyano; R₆ is benzene optionally substituted by 1 to 3 radicals independently selected from (C₁-C₆)alkyl; and X is selected from O,S and NH.

A second aspect of the present invention relates to a compound obtainable by the process according to the first aspect of the invention.

Finally, the third aspect of the invention relates to the use of the compound according to the second aspect of the invention as catalyst.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

In the context of the invention, the term "nanoparticle" refers to a particle with at least two dimensions at the nanoscale, particularly with all three dimensions at the nanoscale, where the nanoscale is the range about 0.6 nm to about 100 nm. Particularly, when the nanoparticle is substantially rod-shaped with a substantially circular cross-section, such as a nanowire or a nanotube, the "nanoparticle" refers to a particle with at least two dimensions at the nanoscale, these two dimensions being the cross-section of the nanoparticle.

The term "obtainable" and "obtained" have the same meaning and are used interchangeably. In any case, the expression "obtainable" encompasses the expression "obtained".

The term "size" refers to a characteristic physical dimension. For example, in the case of a nanoparticle that is substantially spherical, the size of the nanoparticle corresponds to the diameter of the nanoparticle. In the case of a nanoparticle that is substantially rod-shaped with a substantially circular cross-section, such as nanowire or a nanotube, the size of the nanoparticle corresponds to the diameter of the cross-section of the nanoparticle. In the case of a nanoparticle that is substantially box-shaped, such as a nanocube, a nanobox, or a nanocage, the size of the nanoparticle corresponds to the maximum edge length. When referring to a set of nanoparticles as being of a particular size, it is contemplated that the set of nanoparticles can have a distribution of sizes around the specified size. Thus, as used herein, a size of a set of nanoparticles can refer to a mode of a distribution of sizes, such as a peak size of the distribution of sizes.

The term "porous solid support" refers to a particle such as for example alumina or silica that has pores within which moisture or other particles can be adsorbed on the surface or/and in the cavities of the pores of the support.

The term "porous carbon" refers to a carbonaceous material that comprises at least 50 atom % carbon, having a porosity equal to or higher than 5 vol % of the weight of the material. Examples of porous carbon suitable for the present invention include carbon nanotubes, and carbides-derived carbons.

The term "porosity" refers to the percentage of total volume of the material that is occupied by voids.

The term "aromatic compound" or "a compound having one or two aromatic or heteroaromatic rings" have the same meaning and are used interchangeably. They refer to a compound comprising a mono or polycyclic planar molecule having 4n+2 electrons delocalized into an array of p orbitals; being n an integer selected from 1 to 2.

The term "catalytically effective amount" refers to the fact that the amount of catalyst is much smaller than the stoichiometric amounts of either starting materials. The amount is expressed as percentage calculated as the ratio of the number of ruthenium atoms comprised in the catalyst in relation to the number of molecules of aromatic compound.

The term "oxygen-free atmosphere" refers to an atmosphere of a set volume of gas that includes less than about one volume percent oxygen, based on the total volume of the gas in the atmosphere.

The term "solvent-free process" refers to a process that is carried out in the absence of solvent, with the neat substrate.

The term "ligand" refers to a molecule that binds to a superficial surface metal atom through formal donation of one or more of the ligand's electron pairs. There exist different natures of metal-ligand bonding. The most common one between the ligand and the metal is a dative bond, wherein the electron donor atom provides two electrons to the metal atom.

The terms "turnover number" or "TON" have the same meaning and are used interchangeably. They refer to the number of moles of substrate that a mole of catalyst can convert during a certain unit of time. This term usually refers to the point where the hydrogenation reaction is finished, either because the hydrogen has been consumed, the starting materials have been totally converted into the corresponding final compound or because the catalyst has been deactivated. TON is calculated as the number of moles of consumed hydrogen (*n*_{H2}) divided by the number of moles of metal catalyst (*n*_{cat}).

The terms "turnover frequency" or "TOF" have the same meaning and are used interchangeably. They refer to the turnover per unit of time under turnover conditions. The TOF can be calculated by dividing the TON by the time period, in seconds, over which the TON was measured. The TOF is usually expressed in h⁻¹.

The term "turnover conditions" refers to the conditions in which the catalytic hydrogenation takes place, such as for example pressure, temperature, and amount of catalyst.

The term "alkane" refers to a saturated, branched or linear hydrocarbon which contains the number of carbon atoms specified in the description or claims. Examples include but are not limited to methane, ethane, propane, isopropane, butane, isobutane, sec-butane, tert-butane, pentane and hexane.

The term alkyl refers to a saturated straight, or branched hydrocarbon chain which contains the number of carbon atoms specified in the description or claims. Examples include the group methyl, ethyl, propyl, isopropyl, butyl, isobutyl, sec-butyl, tert-butyl, n-pentyl, and n-hexyl.

The term halogen, also referred to as halo, refers to fluoro, chloro, bromo or iodo.

The term (C₁-C₆)haloalkyl refers to a group resulting from the replacement of one or more hydrogen atoms from a (C₁-C₆)alkyl group with one or more halogen atoms, which can be the same or different. Examples include, among others, trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, and nonafluorobutyl.

The term (C₁-C₆)alkoxy refers to an alkoxy group having from 1 to 6 carbon atoms, the alkyl moiety having the same meaning as previously defined. Examples include, among others, methoxy, ethoxy, propoxy, isopropoxy, tert-butoxy, pentoxy and hexyloxy.

The term (C₁-C₆)haloalkoxy refers to a group resulting from the replacement of one or more hydrogen atoms from a (C₁-C₆)alkoxy group with one or more halogen atoms, which can be the same or different.

The term (C₁-C₆)alkylcarbonyl refers to a saturated straight or branched alkyl chain which contains from 1 to 6 carbon atoms, where the carbon atom is appended to the alkyl chain through a carbonyl group.

The term (C₁-C₆)alkoxycarbonyl refers to a saturated straight or branched alkyl chain which contains from 1 to 6 carbon atoms, where the carbon atom is appended to the alkyl chain through an oxycarbonyl group, thus forming an ester.

The term (C₃-C₇)cycloalkyl refers to a saturated carbocyclic ring containing from 3 to 7 carbon atoms, and being the ring optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, and (C₁-C₆)alkoxycarbonyl.

The term (C₃-C₇)cycloalkenyl refers to a carbocyclic ring containing from 3 to 7 carbon atoms and that also contains one or two double bonds, and being the ring optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, and (C₁-C₆)alkoxycarbonyl.

The term known "aryl" refers to a ring system with 1-3 rings which contains the number of carbon atoms specified in the description or claims, the rings being saturated, partially unsaturated, or aromatic; and being fused, bridged, or can contain different types of fusion; being at least one of the rings an aromatic ring; and the ring system being optionally substituted by one or more radicals independently selected from the group consisting of (C₁-C₆)alkyl, (C₁-C₆)haloalkyl, (C₁-C₆)alkoxy, nitro, cyano, and halogen.

The term known "heteroaryl" refers to a ring system with 1-3 rings which contains the number of carbon atoms specified in the description or claims, wherein one or more of the ring members, preferably 1, 2, 3, 4 ring members, is selected from N, NH, O, and S; the rings being saturated, partially unsaturated, aromatic; and being fused, bridged, or can contain different types of fusion; being at least one of the rings an aromatic ring; and the ring system being optionally substituted by one or more radicals independently selected from the group consisting of (C₁-C₆)alkyl, (C₁-C₆)haloalkyl, (C₁-C₆)alkoxy, nitro, cyano, and halogen.

For the purposes of the present invention, in "fused" rings the fusion occurs through one bond which is common to two adjoining rings; and in "bridged" rings the fusion occurs through a sequence of atoms (bridgehead) which is common to two rings.

The term "alkenyl" refers to a branched or linear alkyl chain which contains the number of carbon atoms specified in the description or claims and that also contains one or two double bonds. Examples include, among others, ethenyl, 1-propen-1-yl, 1-propen-2-yl, 3-propen-1-yl, 1-buten-1-yl, 1-buten-2-yl, 3-buten-1-yl, 3-buten-2-yl, 2-buten-1-yl, 2-buten-2-yl, 2-methyl-1-propen-1-yl, 2-methyl-2-propen-1-yl, 1,3-butadien-1-yl, and 1,3-butadien-2-yl.

The term "alkynyl" refers to refers to a branched or linear alkyl chain which contains the number of carbon atoms specified in the description or claims and that also contains one or two triple bonds.

The term "known" ring system as used herein refers to a ring system which is chemically feasible and is known in the art and so intends to exclude those ring systems that are not chemically possible.

As mentioned above, an aspect of the present invention refers to a process for the preparation of the phosphine-ligated ruthenium nanoparticles supported in a porous solid support by reacting a complex of Ru(0) with hydrogen under mild conditions, followed by stabilizing the ruthenium nanoparticles thus formed with the phosphine ligand of formula (I); and supporting the phosphine-ruthenium nanoparticles on a porous solid support.

In comparison with the catalysts of the prior art, the catalyst of the present invention has a higher catalytic activity for the hydrogenation of aromatic compounds, is more stable than the unsupported catalyst, and is more easily recovered and reused without loss of activity.

The efficiency of the reaction is measured by the values of TON obtained, and the relative amount of the resulting hydrogenated cyclic saturated or partially unsaturated compound. Without being bound to theory, it is believed that the nature and amount of the phosphine ligand of formula (I), in combination with the specific reaction conditions of the process for the preparation of nanoparticles and the interaction of the ligated ruthenium nanoparticles on the porous solid support, gives nanoparticles with a reduced particle size, and the appropriate number of reaction free-sites on their surface which allows a high efficiency in the catalytic reaction and improved stability of the catalyst.

The particle size of the phosphine-ruthenium nanoparticles supported on a porous solid support of the present invention is comprised of from 0.7 to 3 nm; in a preferred embodiment the particle size is comprised of from 1 to 3 nm; preferably the particle size is comprised of from 1 to 2 nm.

The amount of ruthenium in the ligated supported-catalyst of the invention is comprised of from 0.1 to 15% by weight. This amount of ruthenium refers to the weight percent of ruthenium contained in the ruthenium nanoparticles supported on the porous solid support of the first aspect of the invention. The amount of ruthenium can vary depending on the pores size of the solid support and the particle size of the nanoparticles used. When the amount of ruthenium is higher than 15% by weight of the total weight of the supported nanoparticles (outside the scope of the present invention) the catalytic activity of the nanoparticles decreases due to the loss of available active surface on the nanoparticle by the agglomeration of the nanoparticles within the solid support; while when the amount of ruthenium is less than 0.1 % by weight of the total weight of the supported nanoparticles (outside the scope of the present invention) the catalytic activity of the nanoparticles decreases due to low availability of the nanoparticles in the solid support. In a preferred embodiment, the amount of ruthenium in ligated supported-catalyst of the invention is comprised of from 0.1 to 10% by weight; more preferably comprised of from 0.1 to 5% by weight.

Step (a) of the process for the preparation of the catalyst according to the first aspect of the present invention comprises the preparation of the phosphine-ruthenium nanoparticles by reacting a solution of the ruthenium (0) complex selected from the group consisting of [Ru(diene)(triene)], [Ru(R₆)(diene)], and Ru₃(CO)₁₂ with hydrogen in the presence of a solvent and of a phosphine ligand of formula (I) under oxygen-free atmosphere.

In a preferred embodiment, the ruthenium (0) complex used in Step (a) is selected from the group consisting of Ru₃(CO)₁₂, Ru(benzene)(cyclohexa-1,3-diene), Ru(cycloocta-1,5-diene) (cycloocta-1,3,5-triene), and Ru(cycloocta-1,3-diene) (cycloocta-1,3,5-triene), preferably the ruthenium complex in Step (a) is Ru(cycloocta-1,5-diene)(cycloocta-1,3,5-triene).

In a particular embodiment, the process for preparing a compound comprising ruthenium nanoparticles supported on a porous support of the present invention, further comprises a previous step of transforming a ruthenium (II) complex or alternatively a ruthenium (III) complex into the ruthenium (0) complex as defined above. It is known in the state of the art that this transformation can be carried out in the presence of a reducing agent (cf. Anthony F. Hill et al. "Bis(methimazoyl)silyl complexes of Ruthenium". Organometallics. 2010, vol. 29, pp. 1026-1031; International patent application number WO2011057780; André Bauer, et al. "Efficient Synthesis of Ruthenium (II) η5-dienyl compounds starting from Di-µ-chlorodichloro-bis[(1-3η:6-8η)-2,7-dimethyloctadienediyl]diruthenium(IV). Versatile precursors for enentioselective hydrogenation catalysts". Organometal·lics. 2000, vol 19, pp. 5471-5476; and Xiaozhong liu, et al. "Polyether phosphate for hydroformylation of higher olefins in non-aqueous system and catalyst recovery "Journal of Organometallic Chemistry. 2002, vol 654(1-2), pp. 83-90). This process is typically carried out by reacting ruthenium (III) or ruthenium (II) halide salt with an appropriate ligand, such as for example cyclooctadiene, cyclohexadiene, and CO, optionally in the presence of a reducing agent such as for example zinc dust.

In an embodiment of the invention, in the phosphine ligand of formula (I) as defined above R₁ is P(R₅)(R₅'); R₂ is H or (C₁-C₁₂)alkyl; and X is O. In another embodiment of the invention, in the phosphine ligand of formula (I) as defined above R₅ and R₅' are equal, being selected from the group consisting of (C₁-C₆)alkyl, (C₃-C₇) cycloalkyl, (C₃-C₇) cycloalkenyl, tolyl and phenyl; preferably, R₅ and R₅' are equal, being phenyl or cyclohexyl; more preferably phenyl.

In an alternative of the phosphine ligand of formula (I) defined above used in Step (a) of the process of the invention, R₃ and R₄ in combination with the adjacent atoms form a 5 to 14-membered ring system, partially unsaturated or aromatic, which comprises from one to three 5 to 6-membered rings, the members of any of the rings being independently selected from C, CH, CH₂, O, N, and NH; being any of the rings optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, (C₁-C₆)alkoxycarbonyl, nitro, cyano, and P(R₅)(R₅').

In a preferred embodiment of the invention, the phosphine ligand (I) is selected from the compound of formula (IA), (IB) and (IC) 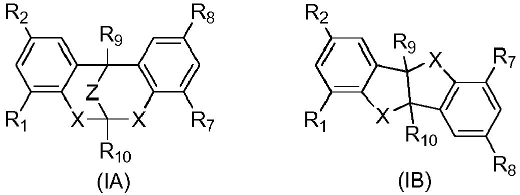 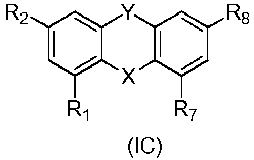 wherein: R₇ and R₈ are independently selected from the group consisting of H, (C₁-C₆)alkyl, (C₁-C₆)alkoxy, and P(R₅)(R₅'), wherein when one of R₇ or R₈ is P(R₅)(R₅'), then the other one of R₇ or R₈ is H, (C₁-C₆)alkyl, or (C₁-C₆)alkoxy; R₉ and R₁₀ are independently selected from the group consisting of H, and (C₁-C₆)alkyl; Y is CH₂ or C((C₁-C₃)alkyl)₂; and Z is a (CH₂)ₙ diradical, being n an integer comprised of from 0 to 3.

In a more preferred embodiment, the phosphine ligand (IA) is selected from the compound of formula (IA') and (IA")

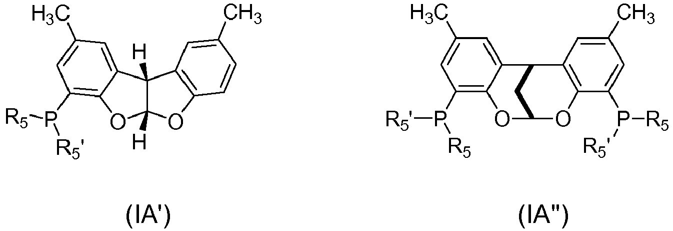

Preferably, the phosphine ligand of formula (IA') is ((5a*R*,10b*S*)-2,9-dimethyl-5a,10b-dihydrobenzofuro[2,3-b]benzofuran-4-yl)diphenylphosphine wherein R₅ and R₅' are phenyl, corresponding to the phosphine ligand 4 of Table 1, and having the following formula 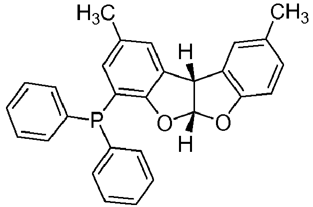 In another preferred embodiment, the phosphine ligand of formula (IA") is ((6S,12S)-2,10-dimethyl-12H-6,12-methanodibenzo[d,g][1,3]dioxocine-4,8-diyl)bis(diphenylphosphine) wherein R₅ and R₅' are phenyl, corresponding to phosphine ligand 2 of Table 1, and having the following formula 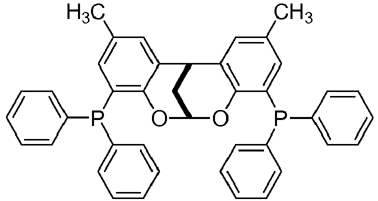 In another alternative process of the invention in the phosphine ligand of formula (I) as defined above used in Step (a) R₃ is (C₁-C₆)alkyl, and R₄ is H or (C₁-C₆)alkyl; preferably, R₁ is P(R₅)(R₅'); R₂ and R₄ are independently selected from the group consisting of H and (C₁-C₆)alkyl; and R₃ is (C₁-C₆)alkyl; more preferably, the phosphine ligand of formula (I) is (2-methoxyphenyl)diphenylphosphine wherein R₅ and R₅' are phenyl, corresponding to phosphine ligand 6 of Table 1, and having the following formula

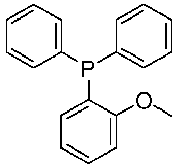

In a preferred embodiment, the molar ratio between the ruthenium (0) complex and the phosphine ligand of formula (I) used in Step (a) of the process for preparing the catalyst of the invention is comprised of from 1:0.05 to 1:0.4. When the molar ratio is higher than 1:0.4 (outside the scope of the invention), Step (a) provides organometallic homogeneous coordination compounds, while when the molar ratio is less than 1:0.05 (outside the scope of the invention) Step (a) provides unstable compounds prompt to aggregation. In a preferred embodiment the molar ratio between the ruthenium (0) complex and the phosphine ligand of formula (I) is comprised of from 1:0.1 to 1:0.3; more preferably, the molar ratio is comprised from 1:0.1 to 1:0.2; preferably, the molar ratio is 1:0.2.

In another preferred embodiment, when carrying out Step (a) of the process of the invention, the concentration of the ruthenium (0) complex is comprised of from 0.6 to 10 mM; preferably, the concentration is comprised of from 2 to 5 mM; more preferably, the concentration is about 3 mM.

Step (a) of the process for the preparation of the catalyst of the first aspect of the present invention is carried out at a temperature comprised of from -40 to 60 °C; preferably the temperature is comprised of from 15 to 35 °C; more preferably, the temperature is comprised of from 20 to 30 °C.

Step (a) of the process for the preparation of the catalyst of the first aspect of the present invention is carried out under a hydrogen pressure comprised of from 1 to 6 bar, preferably under a hydrogen pressure comprised of from 2 to 4 bar. More preferably, Step (a) of the process for the preparation of the catalyst of the first aspect of the present invention is carried out under a hydrogen pressure of 3 bar.

Step (a) of the process for the preparation of the catalyst of the invention is carried out in the presence of a solvent, being the phosphine ligand of formula (I) and the ruthenium (0) complex soluble in the solvent under the reaction conditions of Step (a). A suitable solvent for Step (a) of the present invention is selected from the group consisting of (C₅-C₁₂)alkane, (C₅-C₇)cycloalkane 1,4- dioxane, and tetrahydrofuran; preferably, the solvent is tetrahydrofuran.

In a preferred embodiment, Step (a) for the preparation of the catalyst of the invention comprises reacting Ru(cycloocta-1,3-diene)(cycloocta-1,3,5-triene) as a ruthenium (0) complex, in the presence of a phosphine ligand of formula (I) as defined above with hydrogen under an oxygen-free atmosphere and in tetrahydrofuran; wherein: the molar ratio between the ruthenium (0) complex and the phosphine ligand of formula (I) is comprised of from 1:0.1 to 1:0.2; the concentration of the ruthenium (0) complex is comprised of from 2 to 5 mM; and the reaction is carried out at a temperature comprised of from 15 to 35 °C, and under a hydrogen pressure comprised of from 2 to 4 bar.

Preferably, Step (a) for the preparation of the catalyst of the invention comprises reacting Ru(cycloocta-1,3-diene)(cycloocta-1,3,5-triene) as a ruthenium (0) complex, in the presence of a phosphine ligand of formula (I) as defined above with hydrogen under an oxygen-free atmosphere and in tetrahydrofuran; wherein: the molar ratio between the ruthenium (0) complex and the phosphine ligand of formula (I) is 1:0.2; the concentration of the ruthenium (0) complex is about 3 mM; and the reaction is carried out at a temperature comprised of from 20 to 30 °C, and under a hydrogen pressure of 3 bar.

In a preferred embodiment, Step (a) for the preparation of the catalyst of the invention comprises reacting Ru(cycloocta-1,3-diene)(cycloocta-1,3,5-triene) as a ruthenium (0) complex, in the presence of a phosphine ligand selected from the group consisting of (IA), (IB), and (IC) with hydrogen under an oxygen-free atmosphere and in tetrahydrofuran; wherein: the molar ratio between the ruthenium (0) complex and the phosphine ligand of formula (I) is comprised of from 1:0.1 to 1:0.2; the concentration of the ruthenium (0) complex is comprised from 2 to 5 mM; and the reaction is carried out at a temperature comprised of from 15 to 35 °C, and under a hydrogen pressure comprised of from 2 to 4 bar; more preferably the phosphine ligand is selected from (IA'), and (IA"); preferably, the phosphine ligand (IA') is ((5a*R*,10b*S*)-2,9-dimethyl-5a,10b-dihydrobenzofuro[2,3-b]benzofuran-4-yl)diphenylphosphine wherein R₅ and R₅' are phenyl.

In a preferred embodiment, Step (a) for the preparation of the catalyst of the invention comprises reacting Ru(cycloocta-1,3-diene)(cycloocta-1,3,5-triene) as a ruthenium (0) complex, in the presence of a phosphine ligand selected from the group consisting of (IA), (IB), and (IC) with hydrogen under an oxygen-free atmosphere and in tetrahydrofuran; wherein: the molar ratio between the ruthenium (0) complex and the phosphine ligand of formula (I) is 1:0.2; the concentration of the ruthenium (0) complex is about 3 mM; and the reaction is carried out at a temperature comprised of from 20 to 30 °C, and under a hydrogen pressure of 3 bar; more preferably the phosphine ligand is selected from (IA'), and (IA"); preferably, the phosphine ligand (IA') is ((5a*R*,10b*S*)-2,9-dimethyl-5a,10b-dihydrobenzofuro[2,3-b]benzofuran-4-yl)diphenylphosphine wherein R₅ and R₅' are phenyl.

In a preferred embodiment, Step (a) for the preparation of the catalyst of the invention comprises reacting Ru(cycloocta-1,3-diene)(cycloocta-1,3,5-triene) as a ruthenium (0) complex, in the presence of a phosphine ligand of formula (I) is that wherein R₃ is (C₁-C₆)alkyl, and R₄ is H or (C₁-C₆)alkyl with hydrogen under an oxygen-free atmosphere and in tetrahydrofuran; wherein: the molar ratio between the ruthenium (0) complex and the phosphine ligand of formula (I) is comprised of from 1:0.1 to 1:0.2; the concentration of the ruthenium (0) complex is comprised from 2 to 5 mM; and the reaction is carried out at a temperature comprised of from 15 to 35 °C, and under a hydrogen pressure comprised of from 2 to 4 bar; more preferably the phosphine ligand of formula (I) is (2-methoxyphenyl)diphenylphosphine.

In a preferred embodiment, Step (a) for the preparation of the catalyst of the invention comprises reacting Ru(cycloocta-1,3-diene)(cycloocta-1,3,5-triene) as a ruthenium (0) complex, in the presence of a phosphine ligand of formula (I) is that wherein R₃ is (C₁-C₆)alkyl, and R₄ is H or (C₁-C₆)alkyl with hydrogen under an oxygen-free atmosphere and in tetrahydrofuran; wherein: the molar ratio between the ruthenium (0) complex and the phosphine ligand of formula (I) is 1:0.2; the concentration of the ruthenium (0) complex is about 3 mM; and the reaction is carried out at a temperature comprised of from 20 to 30 °C, and under a hydrogen pressure of 3 bar; more preferably the phosphine ligand of formula (I) is (2-methoxyphenyl)diphenylphosphine.

Step (b) of the process for the preparation of the catalyst according to the first aspect of the present invention comprises supporting the phosphine-ruthenium nanoparticles obtained in Step (a) on a porous solid support by reacting the nanoparticle with the solid support under an oxygen-free atmosphere and in the presence of a solvent; wherein the weight of solid support per mole of ruthenium in the ruthenium (0) complex used in Step (a) is comprised of from 0.5 to 10 g; wherein the reaction is carried out at a temperature comprised of from -40 to 60 °C.

Step (b) of the process for the preparation of the catalyst of the invention is carried out in the presence of solvent. A suitable solvent for Step (b) of the present invention is selected from the group consisting of (C₅-C₁₂)alkane, (C₅-C₇)cycloalkane 1,4-dioxane, and tetrahydrofuran. Preferably, the solvent used in Step (b) is tetrahydrofuran.

In a preferred embodiment, the solvent used in Step (a) and Step (b) is the same and is selected from the group consisting of (C₅-C₁₂)alkane, (C₅-C₇)cycloalkane 1,4- dioxane, and tetrahydrofuran; preferably, the solvent is tetrahydrofuran.

In Step (b) of the process for the preparation of the catalyst of the invention, the weight of solid support per mole of ruthenium in the ruthenium (0) complex used in Step (a) is comprised of from 0.5 to 10 g, preferably the weight of solid support per mole of ruthenium in the ruthenium (0) complex used in Step a) is comprised of from 1 to 10 g.

Step (b) of the process for the preparation of the catalyst of the invention is carried out at a temperature comprised of from -40 to 60 °C; preferably, the temperature is comprised of from 15 to 35 °C; more preferably the temperature is comprised of from 20 to 30 °C.

In a particular embodiment, the process of the invention as defined above is carried out without isolation of the ruthenium nanoparticles obtained in Step (a). It means that the process is a one-pot reaction wherein the nanoparticles obtained in Step (a) are directly contacted with the porous solid support, removing the hydrogen, and introducing an inert gas to obtain the supported ruthenium nanoparticles of the invention.

In comparison with the catalysts of the prior art, a huge range of porous solid supports can be used in Step (b). This is advantageous because the catalyst of the invention is more versatile than the ones known in the state of the art. Thus, in a particular embodiment, the solid support used in Step (b) of the process of preparing the catalyst according to the first aspect of the invention can be any suitable solid support used in the state in the art for supporting metallic nanoparticles. Examples of suitable solid support for the present invention can be, among others, silica, alumina, magnesia, ceria, silicon carbide, hydroxyapatite, porous carbon, zeolite, and titanium oxide. In a preferred embodiment, the porous solid support is a mesoporous solid support. Preferably, the porous solid support is selected from the group consisting of silica, alumina and silicon carbide; more preferably, the porous solid support is selected from alumina and silica.

A compound obtainable by the reaction of the ruthenium (0) complex with hydrogen in the presence of the phosphine ligand of formula (I); followed by the supporting of the nanoparticles thus obtained on a porous solid support as described in the first aspect of the invention is also an object of the present invention. As said above, the compound "obtainable by" the process of the invention is more stable and more efficient for the hydrogenation of aromatic compounds than the ruthenium catalysts of the state of the art.

As said above, the compound obtainable by the process of the first aspect of the invention can be used as catalyst. Thus, the third aspect of the invention refers to the use of the compound obtainable by the process of the first aspect of the invention as catalyst.

As said above, the compound obtainable by the process of the first aspect of the invention can be used as catalyst for the hydrogenation of aromatic compounds. Thus, the third aspect of the invention refers to the use of the compound obtained by the process of the first aspect of the invention for hydrogenating a compound having one or two aromatic or heteroaromatic rings into the corresponding cyclic saturated or partially unsaturated compound which comprises reacting the compound having one or two aromatic or heteroaromatic rings with hydrogen in the presence of an effective amount of the compound as defined in the second aspect of the invention, under an oxygen-free atmosphere, wherein the hydrogenating reaction is carried out at a temperature comprised of from 15 to 120 °C, and under a hydrogen pressure comprised of from 1 to 200 bar.

This aspect can be also formulated as a process for hydrogenating a compound having one or two aromatic or heteroaromatic rings into the corresponding cyclic saturated or partially unsaturated compound which comprises reacting the compound having one or two aromatic or heteroaromatic rings with hydrogen in the presence of an effective amount of the compound as defined in the second aspect of the invention, under an oxygen-free atmosphere wherein the reaction is carried out at a temperature comprised of from 15 to 120 °C, and under a hydrogen pressure comprised of from 1 to 200 bar.

The use of the catalyst of the invention in combination with the specific conditions of hydrogenation reaction allows having high turnover numbers and conversion of the total or partial hydrogenation of the aromatic compounds into the non-aromatic cyclic compounds.

The stability of the catalyst supported on the porous solid support under the reaction conditions of the hydrogenation process facilitates the recycling of the catalyst. Thus, in a preferred embodiment, the process for hydrogenating aromatic compounds as defined above further comprises an additional step of separating the catalyst from the reaction mixture, such as for example by filtration. The catalyst of the invention is also stable even if the process of the invention is stopped, and the catalyst is exposed to air. That means that the catalyst can be directly reused, without a prior activation step in subsequent hydrogenation reactions retaining the initial catalytically activity even after 10 reaction runs.

It is advantageous because the process can be carried out in a continuous way by continuously adding starting materials and hydrogen to a reaction mixture or reaction chamber containing the catalyst of the present invention. Thus, in a preferred embodiment, the hydrogenation process of the invention is a continuous process.

A wide variety of aromatic compounds can be used as a starting material for the preparation of the non-aromatic cyclic compound. In an embodiment of the invention, the aromatic compound used as starting material in the hydrogenation process of the invention is the compound of formula (II) 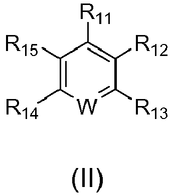 wherein: W is CH or N; R₁₁, R₁₂, R₁₃, R₁₄, and R₁₅ are independently selected from the group consisting of H, (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; or alternatively, R₁₁ and R₁₂, or R₁₂ and R₁₃ in combination with the adjacent atoms form a 5 to 6 membered ring, partially unsaturated or aromatic; the members of the rings being independently selected from C, CH, CH₂, O, S, N, and NH; being the rings optionally substituted by one or more radicals independently selected from the group consisting of (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, CO-NR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from H and (C₁-C₆)alkyl.

In an embodiment of the invention, the aromatic compound used as starting material in the hydrogenation process of the invention is the compound of formula (II) wherein: W is CH or N; R₁₁, R₁₂, R₁₃, R₁₄, and R₁₅ are independently selected from the group consisting of H, (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, - COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from H and (C₁-C₆)alkyl.

In a preferred embodiment, the aromatic compound used as starting material in the hydrogenation process of the invention is the compound of formula (II) wherein: W is CH; R₁₁, R₁₂, R₁₃, R₁₄, and R₁₅ are independently selected from the group consisting of H, (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from H and (C₁-C₆)alkyl. More preferably, W is CH; and R₁₁, R₁₂, R₁₃, R₁₄, and R₁₅ are independently selected from H, and -COO-(C₁-C₁₂)alkyl; preferably, W is CH; and R₁₁, R₁₂, R₁₃, R₁₄, and R₁₅ are H.

In an alternatively preferred embodiment, the aromatic compound used as starting material in the hydrogenation process of the invention is the compound of formula (II) wherein: W is N; R₁₁, R₁₂, R₁₃, R₁₄, and R₁₅ are independently selected from the group consisting of H, (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, - COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from H and (C₁-C₆)alkyl. More preferably, W is N; and R₁₁, R₁₂, R₁₃, R₁₄, and R₁₅ are independently selected from H, and -COO-(C₁-C₁₂)alkyl ; preferably, W is N; and R₁₁, R₁₂, R₁₃, R₁₄, and R₁₅ are H.

In another preferred embodiment, the aromatic compound used as starting material in the hydrogenation process of the invention is the compound of formula (II) wherein: W is CH; R₁₁ and R₁₂, or R₁₂ and R₁₃ in combination with the adjacent atoms form a 5 to 6 membered ring, partially unsaturated or aromatic; the members of the rings being independently selected from C, CH, CH₂, O, S, N, and NH; being the rings optionally substituted by one or more radicals independently selected from the group consisting of (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from H and (C₁-C₆)alkyl.

More preferably, the aromatic compound used as starting material in the hydrogenation process of the invention is the compound of formula (II) wherein: W is CH; R₁₁ and R₁₂, or R₁₂ and R₁₃ in combination with the adjacent atoms form a 5 to 6 membered ring, partially unsaturated or aromatic; the members of the rings being independently selected from C, CH and CH₂; being the rings optionally substituted by one or more radicals independently selected from the group consisting of (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, - COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from H and (C₁-C₆)alkyl.

The hydrogenation reaction of the present invention can be carried out at a temperature comprised of from 15 to 120 °C; preferably, the temperature is comprised of from 80 to 110 °C; more preferably, the temperature is 100 °C.

In another particular embodiment, the hydrogenation process of the present invention is carried out under a hydrogen pressure comprised of from 1 to 200 bar, preferably the hydrogen pressure is comprised of from 20 to 120 bar; more preferably the hydrogen pressure is comprised of from 30 to 80 bar.

The hydrogenation process of the invention for the preparation of the non-aromatic cyclic compound can be carried out in the presence of an organic solvent or without solvent. In a preferred embodiment, the process is carried out without solvent, at a temperature comprised of from 80 to 110 °C, and at a pressure comprised of from 30 to 80 bar.

A wide variety of organic solvents and their mixtures can be used in the hydrogenation of aromatic compounds including non-aromatic aprotic or non-aromatic protic solvents. Examples of appropriate solvents include, among others, (C₅-C₇)alkanes, cyclohexane, tetrahydrofuran (THF), dichloromethane, chloroform, 1,4-dioxane, ethers, and (C₁-C₄)alkyl alcohol.

In a preferred embodiment, the process for hydrogenating aromatic compounds of the present invention is carried out in solvent-free conditions, i.e. in the absence of solvents, when the aromatic compound of formula (II) is liquid in the conditions of reaction. This is advantageous because the reaction is easier to handle and reduces the danger, toxicity and cost. Solvent-free conditions allows better kinetics since the reaction is performed in a more concentrated medium, and simplification of the separation technique used, such as for example one simple distillation or filtration. Additionally, the absence of solvents reduces the cost of the reaction, as well as a lower environmental impact, due to the fact that less waste is generated. The solvent-free hydrogenation process of the present invention can be considered a "green" process in terms of its environmental impact, uses neat aromatic compound as the starting material and solvent, it requires relatively low energy as it occurs under mild conditions, and it is a catalytic reaction, not stoichometric.

In a preferred embodiment, the hydrogenation process of the present invention is carried out without solvent, at a temperature comprised of from 80 to 110 °C, and at a pressure comprised of from 30 to 80 bar.

In a particular embodiment, the hydrogenation process of the present invention has an aromatic compound conversion equal to or higher than 70%, preferably the conversion is equal to or higher than 75%; more preferably, the conversion is equal to or higher than 90%.

Throughout the description and claims the word "comprises" and variations of the word, are not intended to exclude other technical features, additives, components or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

The following abbreviations are used in the below examples
COD: cycloocta-1,5-diene
COT: cycloocta-1,3,5-triene
THF: tetrahydrofuran

### General considerations

### The materials used as solid supports are the following ones:

Silica gel 60A For Chromatography. SDS (Carlo Erba) P2000027 (Batch number Q1 E0I7221 F) CAS: 7631-86-9. Particle size: 35-70 µm; Aluminum oxide (neutral). SDS (Carlo Erba) P2210017 (Batch number Q0I0672801) CAS: 1344-28-1. Specific area: 90-170 m²/g. Particle size: 63-200 µm;SiC: Silicon carbide. Aldrich 378097 (Lot#MKBG9635V) CAS: 409-21-2. Particle size: 200-450 mesh; CNF: Carbon nanofibers. Aldrich 719803 (Lot#MKBD7136V). Graphitized, platelets (conical). Particle size: D x L = 100 nm x 20-200 µm.

### Example 1: Process for the preparation of the supported phosphine-liqated ruthenium nanoparticle (Rul^{0.2}@Y%S)

Ru(cod)(cot) was dissolved in THF (1ml/mg of Ru(cod)(cot)) containing 0.2 equivalents of the phosphine ligand of formula (I) defined in Table 1 in a closed-pressure bottle. The Parr shaker reactor was then pressurized at room temperature under 3 bars of hydrogen and vigorously shacked overnight. The reaction mixture went from yellow to dark brown in few minutes.

After the selected period of time, the hydrogen pressure was eliminated, the porous support added and the solution stirred under argon for 120 min. Then the solution was filtered and degassed. THF was passed through the supported phosphine-ligated ruthenium nanoparticle until a clean solution was obtained. The obtained supported phosphine-ligated ruthenium nanoparticle in solid form was then dried under high vacuum.

The nomenclature of the supported phosphine-ligated ruthenium nanoparticle (Rul^{0.2}@Y%S) is specified as follows:
I refers to the phosphine ligand of formula (I);
0.2 refers to the number of equivalents of the ligand expressed as moles of ligand of formula (I) per mole of Ru;
Y refers to weight percent of ruthenium used for the preparation of the nanoparticle respective to the solid support that has been used to prepare the supported nanoparticle of the invention.
S refers to the porous support.

The amount of the ruthenium complex, the phosphine ligand of formula (I) used and its quantity, the amount of solid support, as well as the elemental analysis of the obtained supported phosphine-ligated nanoparticles are shown in Table 1:

| Supported phosphine-ligated nanoparticles | | Amount of Ru (0) complex Ru(cod)(cot) (mg) | ligand of formula (I) (mg) | | Amount of solid support (mg) | Elemental analysis of the nanoparticles (%) | | | |
|---|---|---|---|---|---|---|---|---|---|
| Number | Formula | | ligand | Amount (mg) | | Ru⁴ | Si | C | H |
| 1 | Ru4⁰²@1SiO₂ | 13 | 4¹ | 3.5 | 412 | 0.33 | 42.21 | 0.38 | 0.08 |
| 2 | Ru4⁰²@2SiO₂ | 13 | 4¹ | 3.5 | 208 | 0.62 | 42.85 | 0.65 | 0.10 |
| 3 | Ru4u⁰²5SiO₂ | 13 | 4¹ | 3.5 | 83 | 1.12 | 42.19 | 1.62 | 0.21 |
| 4 | Ru4⁰²@10SiO₂ | 13 | 4¹ | 3.5 | 42 | 1.59 | 39.89 | 3.40 | 0.40 |
| 5 | Ru4⁰²@15SiO₂ | 13 | 4¹ | 3.5 | 28 | 2.70 | 38.13 | 5.90 | 0.78 |
| 6 | Ru2⁰²@1SiO₂ | 9 | 2² | 3.5 | 288 | - | - | - | - |
| 7 | Ru2⁰²@5SiO₂ | 9 | 2² | 3.5 | 55 | - | - | - | - |
| 8 | Ru2⁰²@10SiO₂ | 9 | 2² | 3.5 | 26 | - | - | - | - |
| 9 | Ru6⁰²@1SiO₂ | 9 | 6 | 3.5 | 288 | - | - | - | - |
| 10 | Ru6⁰²@5SiO₂ | 9 | 6 | 3.5 | 55 | - | - | - | - |
| 11 | Ru6⁰²@10SiO₂ | 9 | 6 | 3.5 | 26 | - | - | - | - |
| 12 | Ru6⁰²@1Al₂0₃ | 9 | 6 | 3.5 | 288 | - | - | - | - |
| 13 | Ru6⁰²@5Al₂O₃ | 9 | 6 | 3.5 | 55 | - | - | - | - |
| 14 | Ru6⁰²@1SiC | 9 | 6 | 3.5 | 288 | - | - | - | - |
| 15 | Ru6⁰²@5SiC | 9 | 6 | 3.5 | 55 | - | - | - | - |
| 16 | Ru6⁰²@1CNF | 9 | 6 | 3.5 | 288 | - | - | - | - |
| 17 | Ru6⁰²@5CNF | 9 | 6 | 3.5 | 55 | - | - | - | - |

| | | | | | | | | | |
|---|---|---|---|---|---|---|---|---|---|
| ¹ Phosphine ligand 4 of Table 1 corresponds to a compound of formula (IA') wherein R₅ and R₅' are phenyl. ² Phosphine ligand 2 of Table 1 corresponds to a compound of formula (IA") wherein R₅ and R₅' are phenyl. ³ Phosphine ligand 6 of Table 1 corresponds to (2-methoxyphenyl)diphenylphosphine. ⁴ The value of Ruthenium in the elemental analysis corresponds to the weight percent of ruthenium contained in the ruthenium nanoparticles supported on the porous solid support. | | | | | | | | | |

### Example 2: Process for the total hydrogenation of aromatic compounds

The hydrogenation process is as follows:

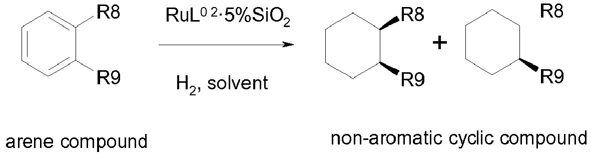

The supported phosphine-ligated nanoparticles obtained in Example 1 were placed in a Hastelloy autoclave. The aromatic compound was added through a liquid injection port in form of: (a) a solution of the solvent described in Table 2 (0.125 M), or (b) as a solvent-free aromatic reagent. The autoclave was pressurized with molecular hydrogen to the reaction conditions of pressure and temperature indicated in Table 2. After the reaction time indicated in Table 2, the autoclave was depressurized. The resulting solution was analyzed by gas chromatography (GC) and nuclear magnetic resonance (NMR) spectroscopy to determine the conversion of aromatic compound into the non-aromatic cyclic compound.

The aromatic compound, the catalyst and solvent used, and the content of ruthenium are shown in Table 2.

The reaction conditions of pressure, temperature, and time, as well as the conversion and values of TOF are shown are shown in Table 3.

**Table 2**

| **Exp.N°** | **R₈/R₉** | **Catalyst** | **Solvent** | **Ru loading** |
|---|---|---|---|---|
| Comparative Ex. 1 | H | Özkar^{(a)} | none | 0,008% |
| Comparative Ex. 2 | H | Ru4^{02(b)} | none | 0,034% |
| Comparative Ex. 3^{(c)} | H | Ru4^{02(b)} | none | 0,032% |
| Ex. 4^{(d)} | H | 3^{(e)} | none | 0,008% |
| Ex. 5 | H | 4^{(e)} | none | 0,005% |
| Ex. 6 | H | 5^{(e)} | none | 0,006% |
| Ex. 7 | H | 12^{(e)} | none | 0,008% |
| Ex. 8 | H | 12^{(e)} | none | 0,009% |
| Comparative Ex. 9 | CO2iNo | Ru4^{02(b)} | none | 0,068% |
| Comparative Ex. 10 | CO2iNo | Ru4^{02(b)} | 50% THF | 0,068% |
| Ex. 11 | CO2iNo | 3^{(e)} | none | 0,082% |

| | | | | |
|---|---|---|---|---|
| ^{(a)} ruthenium catalyst comprising nanozeolite framework ligated Ru(0) nanoclusters described in the Journal of the American Chemical Society, 2010, vol. 132, pp. 6541-6549. ^{(b)} The catalyst Ru4⁰² is an unsupported ruthenium nanoparticles ligated by 0.2 equivalents of the phosphine ligand 4, which corresponds to a compound of formula (IA') wherein R₅ and R₅' are phenyl described in ACS Catalysis, 2012, vol. 2(3), pp. 317-321. ^{(c)} The catalyst Ru4⁰² was recycled and used in subsequent runs. ^{(d)} Supported catalyst 3 of the present invention was recycled and used in subsequent runs. (e) the catalyst number corresponds to the Supported phosphine-ligated nanoparticles number of Table 1 | | | | |

**Table 3**

| **Exp.N°** | **Pressure (bar)** | **Temp. (°C)** | **time (h)** | **Conversion %** | **TOF (h⁻¹)** |
|---|---|---|---|---|---|
| Comparative Ex. 1 | 3 | 25 | 2,25 | 100 | 5430 |
| Comparative Ex. 2 | 3 | 25 | 0,16 | 4 | 1987 |
| Comparative Ex. 3 | 40 | 100 | 0,16 | 72^{(a)} | 42000^{(a)} |
| | | | | 82^{(b)} | 48000^{(b)} |
| | | | | 12^{(c)} | 7000^{(c)} |
| Ex. 4 | 40 | 100 | 0,3 | 77^{(d)} | 99193^{(d)} |
| Ex. 5 | 40 | 100 | 0,47 | 100 | 86214 |
| Ex. 6 | 40 | 100 | 0,71 | 70 | 51260 |
| Ex. 7 | 40^{(e)} | 100 | 1 | 74 | 26946 |
| Ex. 8 | 40 | 100 | 1,1 | 100 | 30345 |
| Comparative Ex. 9 | 50 | 90 | 18 | 100 | 232 |
| Comparative Ex. 10 | 50 | 90 | 2,1 | 100 | 2110 |
| Ex. 11 | 50 | 90 | 1 | 100 | 3362 |

| | | | | | |
|---|---|---|---|---|---|
| ^{(a)} Conversion and value of TOF of the first run. ^{(b)} Conversion and value of TOF of the second run. ^{(c)} Conversion and value of TOF of the third run. ^{(d)} Conversion and value of TOF of the tenth run. ^{(e)} The initial pressure was 40 bar, and the pressure when the reaction was carried out decreased until 25 bar. | | | | | |

The above results of Table 3 show that in comparison with the unsupported Ru4⁰² catalyst known in the state of the art, the supported catalyst which comprises ruthenium ligated with 0.2 equivalents of the phosphine ligand of formula (I) being a phosphine ligand of formula IA', containing from 0.1 to 15% of ruthenium of the present invention (Entries 4-6) allow the total hydrogenation of aromatic compounds with a high conversion and/or with a high efficiency of the catalyst as it is observed by the higher rates (TOF) even when different reaction conditions of temperature, pressure, and time are tested.

The above results of Table 3 also show that:
1. The hydrogenation of aromatic compounds using the known unsupported Ru4^{0.2} catalyst values of turnover (TOF) about 45000 in the first run with a ruthenum loading about 30% (cf. comparative Example 3). In comparison, the turnover values of TOF after the tenth run using the supported catalyst of the present invention are higher (about 99000) even when the nanoparticles has a lower ruthenium loading (0.08%) (cf. Example 4).
2. The known unsupported Ru4^{0.2} catalyst can be recycled for two runs having a loss of efficiency as it is observed in the decrease of the values of TOF(comparative Example 3). In comparison, the supported catalyst of the present invention can be recycled up to ten times without losing efficiency of the catalyst (cf. Example 4).

The above results of Table 3 also show that in comparison with the unsupported Ru4^{0.2} catalyst known in the state of the art, the supported catalyst which comprises ruthenium ligated with 0.2 equivalents of a monophosphine ligand of formula (I), being (2-methoxyphenyl)diphenylphosphine, containing from 0.1 to 15% of ruthenium of the present invention (Entries 7 and 8) allow the total hydrogenation of aromatic compounds with a high conversion and/or with a high efficiency of the catalyst as it is observed by the higher rates (TOF) even when different reaction conditions of temperature, pressure, and time are tested. It is advantageous, because the monophosphine ligand (2-methoxyphenyl)diphenyl-phosphine does not require previous preparation because it is commercially available.

Finally, the above results of Table 3 also show that in comparison with the unsupported Ru4^{0.2} catalyst known in the state of the art (Entries 9, and 10), the supported catalyst which comprises ruthenium ligated with 0.2 equivalents of a phosphine ligand of formula (I), being a phosphine ligand of formula IA', containing from 0.1 to 15% of ruthenium of the present invention (Entry 11) allow the total hydrogenation of aromatic compounds substituted with ester groups with a high conversion and with a high efficiency of the catalyst as it is observed by the higher rates (TOF) even when different reaction conditions of temperature, pressure.

Thus, it is demonstrated that the catalyst of the present invention comprising the ruthenium nanoparticles ligated with the phosphine compound of formula (I) supported on a solid support as defined above is more stable and efficient than the corresponding unsupported colloidal ruthenium nanoparticles for the total hydrogenation of aromatic compounds, being reusable without loss of catalytic activity.

### REFERENCES CITED IN THE APPLICATION

- Ozkar, et al. "Ruthenium (0) nanoclusters stabilized by a nanozeolite framework:isolable, reusable, and green catalyst for the hydrogenation of neat aromatics under mild conditions with the unprecedented catalytic activity and lifetime", Journal of the American Chemical Society, 2010, vol. 132, pp. 6541-6549.
- Piet W.N.M van Leeuwen, et al. "Phosphine stabilized ruthenium nanoparticles: the effect of the nature of the ligand in catalysis", ACS Catalysis, 2012, vol. 2(3), pp. 317-321.
- Montserrat Gómez, et al. "Ruthenium nanoparticles supported on multi-walled carbon nanotubes: highly effective catalytic system for hydrogenation processes", Journal of Molecular Catalysis A: Chemical, 2010, vol. 332, pp. 106-112.
- Anthony F. Hill et al. "Bis(methimazoyl)silyl complexes of Ruthenium". Organometallics. 2010, vol. 29, pp. 1026-1031.
- International patent application number WO2011057780.
- André Bauer, et al. "Efficient Synthesis of Ruthenium (II) η5-dienyl compounds starting from Di-µ-chlorodichloro-bis[(1-3η:6-8η)-2,7-dimethyloctadienediyl]diruthenium(IV). Versatile precursors for enentioselective hydrogenation catalysts". Organometal·lics. 2000, vol 19, pp. 5471-5476.
- Xiaozhong liu, et al. "Polyether phosphate for hydroformylation of higher olefins in non-aqueous system and catalyst recovery "Journal of Organometallic Chemistry. 2002, vol 654(1-2), pp. 83-90.

## Claims

1. A process for preparing a compound comprising ruthenium nanoparticles supported on a porous solid support, wherein the particle size of the ruthenium nanoparticles is comprised of from 0.7 to 3 nm; and the amount of ruthenium in the ruthenium nanoparticles supported on the porous solid support is comprised of from 0.1 to 15% by weight;
the process comprising:
(a) reacting a ruthenium (0) complex selected from the group consisting of [Ru(diene)(triene)], [Ru(R₆)(diene)], Ru₃(CO)₁₂, and a mixture thereof, in the presence of a phosphine ligand of formula (I) or a mixture thereof 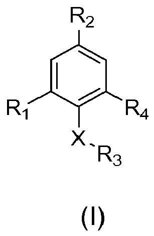 with hydrogen under an oxygen-free atmosphere and in a solvent; wherein: the molar ratio between the ruthenium (0) complex and the phosphine ligand of formula (I) is comprised of from 1:0.05 to 1:0.4; the concentration of the ruthenium (0) complex is comprised of from 0.6 to 10 mM and the reaction is carried out at a temperature comprised of from -40 to 60 °C, and under a hydrogen pressure comprised of from 1 to 6 bar; and
(b) reacting the ruthenium nanoparticles obtained in Step (a) with a porous solid support under an oxygen-free atmosphere and in the presence of a solvent; wherein: the weight of solid support per mole of ruthenium in the ruthenium (0) complex used in Step (a) is comprised of from 0.5 to 10 g; wherein the reaction is carried out at a temperature comprised of from -40 to 60°C;
wherein:
diene is selected from the group consisting of cycloocta-1,5-diene, cycloocta-1,3-diene, norbornadiene, cyclohexa-1,3-diene, and hepta-1,4-diene;
triene is cycloocta-1,3,5-triene;
R₁ and R₂ are independently selected from the group consisting of H, (C₁-C₁₂)alkyl, (C₁-C₆)alkoxy, and P(R₅)(R₅'), wherein one of R₁ or R₂ is P(R₅)(R₅'), and the other of R₁ or R₂ is H, (C₁-C₆)alkyl, or (C₁-C₆)alkoxy;
R₃ is (C₁-C₆)alkyl;
R₄ is H or (C₁-C₆)alkyl; or alternatively
R₃ and R₄ in combination with the adjacent atoms form a 5 to 14-membered ring system, partially unsaturated or aromatic, which comprises from one to three 5 to 6-membered rings, the members of any of the rings being independently selected from C, CH, CH₂, O, N, and NH; being any of the rings optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, (C₁-C₆)alkoxycarbonyl, nitro, cyano, and P(R₅)(R₅');
R₅ and R₅' are independently selected from the group consisting of (C₁-C₆)alkyl, (C₃-C₇)cycloalkyl, (C₃-C₇)cycloalkenyl, (C₆-C₁₄)aryl, and (C₄-C₁₄)heteroaryl; being optionally substituted by one or more radicals independently selected from the group consisting of (C₁-C₆)alkyl, (C₁-C₆)haloalkyl, halogen, (C₁-C₆)alkoxy, (C₁-C₆)alkylcarbonyl, (C₁-C₆)alkoxycarbonyl, nitro, and cyano;
R₆ is benzene optionally substituted by 1 to 3 radicals independently selected from (C₁-C₆)alkyl; and
X is selected from O, S and NH.

2. The process according to claim 1, wherein in the phosphine ligand of formula (I) R₁ is P(R₅)(R₅'); R₂ is H or (C₁-C₁₂)alkyl; and X is O.

3. The process according to any of the claims 1-2, wherein in the phosphine ligand of formula (I) R₅ and R₅' are equal, being selected from the group consisting of (C₁-C₆)alkyl, (C₃-C₇)cycloalkyl, (C₃-C₇)cycloalkenyl, tolyl and phenyl.

4. The process according to any of the claims 1-3, wherein the phosphine ligand (I) is selected from the compound of formula (IA), (IB) and (IC) 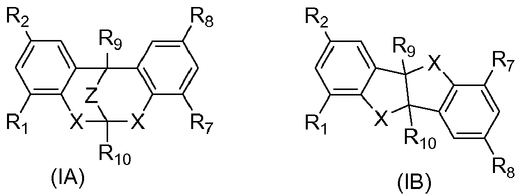 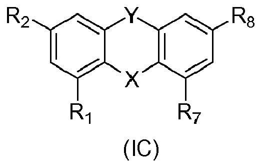 wherein:
R₇ and R₈ are independently selected from the group consisting of H, (C₁-C₆)alkyl, (C₁-C₆)alkoxy, and P(R₅)(R₅'), wherein when one of R₇ or R₈ is P(R₅)(R₅'), then the other one of R₇ or R₈ is H, (C₁-C₆)alkyl or (C₁-C₆)alkoxy;
R₉ and R₁₀ are independently selected from the group consisting of H and (C₁-C₆)alkyl;
Y is CH₂ or C((C₁-C₃)alkyl)₂; and
Z is a (CH₂)ₙ diradical, being n an integer comprised of from 0 to 3.

5. The process according to claim 4, wherein the phosphine ligand of formula (IA) is selected from the compound of formula (IA') and (IA")

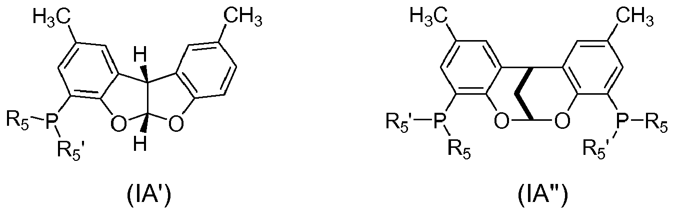

6. The process according to claim 5, wherein the phosphine ligand of formula (IA') is ((5a*R*,10b*S*)-2,9-dimethyl-5a,10b-dihydrobenzofuro[2,3-b]benzofuran-4-yl)diphenylphosphine.

7. The process according to any of the claims 1-3, wherein in the phosphine ligand of formula (I) R₁ is P(R₅)(R₅'); R₂ and R₄ are independently selected from the group consisting of H and (C₁-C₆)alkyl; and R₃ is (C₁-C₆)alkyl.

8. The process according to claim 7, wherein the phosphine ligand of formula (I) is (2-methoxyphenyl)diphenylphosphine.

9. The process according to any of the claims 1-8, wherein the porous solid support is selected from the group consisting of silica, alumina, magnesia, ceria, hydroxyapatite, porous carbon, zeolite, and titanium oxide.

10. The process according to any of the claims 1-9, wherein the Ru(0) complex is selected from the group consisting of Ru(benzene)(cyclohexa-1,3-diene), Ru(cycloocta-1,5-diene)(cycloocta-1,3,5-triene), and Ru(1,3-cycloocta-1,3-diene)(cycloocta-1,3,5-triene).

11. A compound obtainable by the process according to any of the claims 1-10.

12. The use of the compound according to claim 11 as catalyst.

13. The use of the compound according to claim 12 for hydrogenating a compound having one or two aromatic or heteroaromatic rings into the corresponding cyclic saturated or partially unsaturated compound which comprises reacting the compound having one or two aromatic or heteroaromatic rings with hydrogen in the presence of an effective amount of the compound as defined in claim 10, under an oxygen-free atmosphere, wherein the hydrogenating reaction is carried out at a temperature comprised of from 15 to 120 °C, and under a hydrogen pressure comprised of from 1 to 200 bar.

14. The use according to claim 13, wherein the compound having one or two aromatic or heteroaromatic rings is a compound of formula (II): 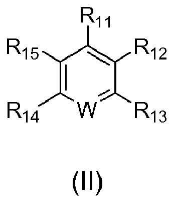 wherein:
W is CH or N;
R₁₁, R₁₂, R₁₃, R₁₄, and R₁₅ are independently selected from the group consisting of H, (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; or alternatively,
R₁₁ and R₁₂, or R₁₂ and R₁₃ in combination with the adjacent atoms form a 5 to 6 membered ring, partially unsaturated or aromatic; the members of the rings being independently selected from C, CH, CH₂, O, S, N, and NH; being the rings optionally substituted by one or more radicals independently selected from the group consisting of (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, CO-NR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and
R₁₆ and R₁₇ are independently selected from H and (C₁-C₆)alkyl.

15. The use according to claim 14, wherein in the compound of formula (II) W is CH; R₁₁, R₁₂, R₁₃, R₁₄, and R₁₅ are independently selected from the group consisting of H, (C₁-C₆)alkyl, halogen, (C₁-C₆)haloalkyl, hydroxy, (C₁-C₆)alkoxy, -CHO, NR₁₆R₁₇, -CO-(C₁-C₆)alkyl, -COO-(C₁-C₁₂)alkyl, -COOH, -CONR₁₆R₁₇, -CO-(C₁-C₆)alkyl-N((C₁-C₆)alkyl)₂, cyano, (C₁-C₁₂)alkenyl, (C₁-C₆)alkyl-CO-(C₁-C₆)alkyl, and (C₁-C₁₂)alkynyl; and R₁₆ and R₁₇ are independently selected from from H and (C₁-C₆)alkyl.
